# EUROPEAN PATENT APPLICATION

(11) **EP 1 806 162 A2**
(43) Date of publication of application: **11.07.2007**
(21) Application number: 07100051.7
(22) Date of filing: 03.01.2007
(51) Int. Cl.: A61N 5/10, G06F 17/00

(54) **System and method for longitudinal patient dosimetry management decision support**

(30) Priority: 06.01.2006 US 327556
(71) Applicant: General Electric Company, Schenectady, NY 12345 (US)
(72) Inventor: Gentles, Thomas A., Algonquin, IL 60102 (US)
(74) Representative: Bedford, Grant Richard

(57) **Abstract**

Certain embodiments of the present invention provide for a system (100, 200) for patient dosimetry management including a processing component (120, 220). The processing component (120, 220) is capable of receiving and/or aggregating dosimetry data from a plurality of information sources (110, 210). Each information source (110, 210) in the plurality of information sources (110, 210) includes dosimetry data for a patient. The processing component (120, 220) is capable of generating patient dosimetry information based at least in part on the aggregated dosimetry data.

## Description

The present invention generally relates to patient dosimetry management. In particular, the present invention relates to a system and method for longitudinal patient dosimetry management decision support.

Medical imaging systems may be used to capture images to assist a physician in making an accurate diagnosis. Images acquired from medical imaging systems allow a physician or other healthcare provider to obtain information about a patient that might otherwise be unavailable except by more invasive techniques. Imaging systems typically include a source and a detector. Energy, such as x-rays, produced by the source travel through an object to be imaged and are detected by the detector. An associated control or image processing system obtains image data from the detector and prepares a corresponding diagnostic image on a display.

Image data may come from a variety of sources. Images may have been generated and/or acquired from one or more imaging sessions and involve different modalities (e.g., ultrasound (US), magnetic resonance (MR), computed tomography (CT), x-ray, positron emission tomography (PET), nuclear, thermal, optical, video, etc.), views, slices, and/or protocols. Images may have originated from a single source or be a result of calculation (e.g., fused or compound images from multiple modalities).

Healthcare environments, such as hospitals or clinics, include clinical information systems, such as hospital information systems (HIS) and radiology information systems (RIS), and storage systems, such as picture archiving and communication systems (PACS). Information stored may include patient medical histories, imaging data, test results, diagnosis information, management information, and/or scheduling information, for example. The information may be centrally stored or divided at a plurality of locations. Healthcare practitioners may desire to access patient information or other information at various points in a healthcare workflow. For example, during surgery, medical personnel may access patient information, such as images of a patient's anatomy, that are stored in a medical information system. Alternatively, medical personnel may enter new information, such as history, diagnostic, or treatment information, into a medical information system during an ongoing medical procedure.

PACS connect to medical diagnostic imaging devices and employ an acquisition gateway (between the acquisition device and the PACS), storage and archiving units, display workstations, databases, and sophisticated data processors. These components are integrated together by a communication network and data management system. A PACS has, in general, the overall goals of streamlining health-care operations, facilitating distributed remote examination and diagnosis, and improving patient care.

Radiation, such as x-rays, may be used both for diagnostic purposes as well as for treatment. Diagnostic purposes may include, for example, medical imaging. As discussed above, medical images are important in making an accurate diagnosis. Treatment purposes may include, for example, radiation therapy to treat patients with cancer. Radiation therapy allows certain cancers to be treated while reducing or eliminating the need for invasive surgery.

Exposure to radiation, such as from medical imaging or radiation therapy, may have health effects. For example, radiation may cause nausea, hair loss, tissue damage, and death. Radiation exposure is cumulative over a person's lifetime. Generally, the amount and duration of exposure to radiation affects the type and/or severity of health effects.

Radiation dosage may be represented in a variety of ways. Examples of units of radiation dosage include grays (Gy), Joules/kilogram (J/kg), Sieverts (Sv), ergs/gram (erg/g) and Roentgens (R). Radiation dosage may be measured as, for example, absorbed dose, effective dose, and equivalent dose. The absorbed dose measures the energy deposited per unit mass of a medium. Absorbed dose may be measured in grays (Gy) or Roentgens Absorbed Dose (rad), for example. The effective dose is calculated using a weighted average of equivalent doses for different body tissues based on the sensitivity of different tissues to radiation. The equivalent dose measures radiation dosage taking into account the biological effects of different kinds of radiation. Effective dose may be measured in Roentgen equivalent man (rem), for example. Different representations of radiation dosage may be preferred depending on the situation. A multiple of the Sievert, the millisievert (mSv) is commonly used to measure the effective dose in diagnostic medical procedures (e.g., X-rays, nuclear medicine, positron emission tomography and computed tomography).

The optimal procedures and/or courses of treatment to perform should be based upon a number of criteria. Criteria to consider include, for example, the particular problem a patient has, the patient's age, gender, and physical condition. Another important criteria that should be considered is the longitudinal patient dosimetry. That is, the radiation dosage history of the patient.

Current RIS and PACS systems are used to manage the protocol specification, execution, and reporting of procedures such as x-ray and CT exams. However, current systems such as RIS and PACS do not have the capability to allow longitudinal patient dosimetry criteria into the process for determining procedures and courses of treatment. Thus, there is a need for a system and method for longitudinal patient dosimetry management decision support.

Certain embodiments of the present invention provide for a system for patient dosimetry management including a processing component. The processing component is capable of receiving and/or aggregating dosimetry data from a plurality of information sources. Each information source in the plurality of information sources includes dosimetry data for a patient. The processing component is capable of generating patient dosimetry information based at least in part on the aggregated dosimetry data.

In an embodiment, the processing component is capable of transforming the dosimetry data into a selected representation. In an embodiment, the processing component is capable of analyzing the patient dosimetry information. In an embodiment, the processing component is capable of providing decision support based at least in part on the patient dosimetry information. In an embodiment, the processing component requests at least part of the dosimetry data from at least one information source in the plurality of information sources. In an embodiment, at least one information source in the plurality of information sources provides at least part of the dosimetry data to the processing component when new dosimetry data is available. In an embodiment, the processing component generates the patient dosimetry information based at least in part on stored dosimetry data. Certain embodiments include a display. The display is capable of displaying at least one of the dosimetry data and the patient dosimetry information.

Certain embodiments of the present invention provide a method for patient dosimetry management including receiving dosimetry data at a processing component and generating patient dosimetry information. The dosimetry data is received from a plurality of information sources. The patient dosimetry information is generated based at least in part on the dosimetry data.

Certain embodiments include utilizing a registry to facilitate communication between the processing component and at least one information source in the plurality of information sources. Certain embodiments include displaying at least one of the patient dosimetry information and the dosimetry data. Certain embodiments include transforming the dosimetry data into a selected representation. Certain embodiments include analyzing the patient dosimetry information. Certain embodiments include providing a recommendation based at least in part on the patient dosimetry information. In an embodiment, the recommendation includes recommending a procedure based at least in part on the patient dosimetry information. In an embodiment, the recommendation includes recommending a course of treatment based at least in part on the patient dosimetry information. In an embodiment, the recommendation includes recommending a radiology procedure protocol based at least in part on the patient dosimetry information.

Certain embodiments of the present invention provide a computer-readable medium including a set of instructions for execution on a computer, the set of instructions including an aggregation routine and a processing routine. The aggregation routine is configured to receive and/or aggregate dosimetry data from a plurality of information systems. The processing routine is configured to generate patient dosimetry information based at least in part on the dosimetry data.

In an embodiment, the processing routine is configured to transform the dosimetry data into a selected representation. In an embodiment, the processing routine is configured to analyze the patient dosimetry information. Certain embodiments include a decision support routine configured to provide decision support based at least in part on the patient dosimetry information.

Various aspects and embodiments of the present invention will now be described in connection with the accompanying drawings, in which:
Figure 1 illustrates a system for patient dosimetry management used in accordance with an embodiment of the present invention.
Figure 2 illustrates a system for patient dosimetry management used in accordance with an embodiment of the present invention.
Figure 3 illustrates a flow diagram for a method for patient dosimetry management used in accordance with an embodiment of the present invention.

The foregoing summary, as well as the following detailed description of certain embodiments of the present invention, will be better understood when read in conjunction with the appended drawings. For the purpose of illustrating the invention, certain embodiments are shown in the drawings. It should be understood, however, that the present invention is not limited to the arrangements and instrumentality shown in the attached drawings.

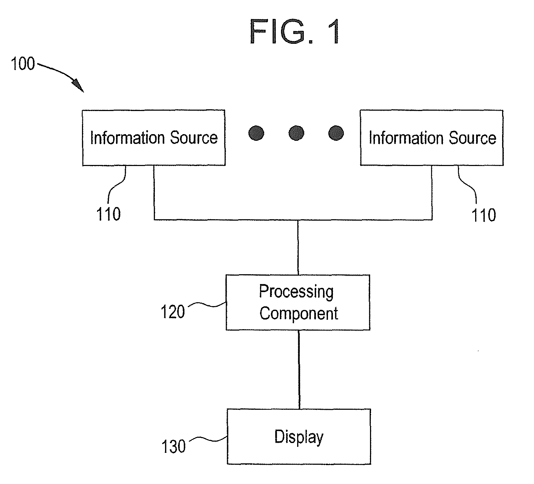

Figure 1 illustrates a system 100 for patient dosimetry management used in accordance with an embodiment of the present invention. The system 100 includes a plurality of information sources 110, a processing component 120, and a display 130. The processing component 120 is in communication with the plurality of information sources 110. The processing component 120 is in communication with the display 130.

Information source 110 includes dosimetry data. Dosimetry data may include, for example, radiation dosage data, procedure data, treatment data, and/or a partial dosimetry history. For example, dosimetry data may include a representation of radiation dosage administered to a patient as part of an x-ray imaging procedure. As another example, dosimetry data may include parameters used in a course of radiation therapy administered to the patient. As another example, dosimetry data may include a partial dosimetry history for a patient that was treated at another clinic. Partial dosimetry history may include, for example, one or more pieces of dosimetry data. For example, partial dosimetry history may include data from several procedures and/or course of treatment. Dosimetry data may be raw data from an information source 110. For example, the dosimetry data may be in a format and/or representation native to the information source 110.

In operation, the processing component 120 receives dosimetry data from one or more information sources 110 in the plurality of information sources. An information source 110 may be one or more of, for example, a PACS, RIS, HIS, acquisition modality, database, control computer, workstation, and/or patient records, for example. For example, dosimetry data may be received from a patient record stored in a HIS. As another example, dosimetry data may be received from an acquisition modality such as a CT scanner.

The processing component 120 may receive dosimetry data over a network, for example. The network may be or may be part of an intranet, the Internet, HIS, RIS, or PACS, for example. For example, an information source 110 and the processing component 120 may be connected to a PACS. The processing component 120 may then receive dosimetry data from the information source 110 through part of the PACS network.

In an embodiment, an information source 110 may be accessed when dosimetry data is needed by the processing component 120 in a "pull" model. That is, the processing component 120 may receive dosimetry data because the processing component 120 requested the dosimetry data from an information source 110. In an embodiment, an information source 110 may provide dosimetry data to the processing component 120 in a "push" model. That is, an information source 110 may send new and/or changed dosimetry data to the processing component 120 when some event and/or change is made to the dosimetry data or new dosimetry data becomes available.

In an embodiment, patient dosimetry information is aggregated at least in part by the processing component 120. For example, dosimetry data such as parameter information for three x-rays may be received at the processing component 120 from one or more information sources 110.

The processing component 120 generates patient dosimetry information based at least in part on the dosimetry data. Patient dosimetry information includes, for example, a dosimetry history for a patient and parameters of procedures and/or treatments for the patient. Patient dosimetry information may also include and/or be based at least in part on transformed dosimetry data and/or patient dosimetry information, results from analyzed dosimetry data and/or other patient dosimetry information, and/or decision support based at least in part on dosimetry data and/or other patient dosimetry information. For example, the processing component 120 may generate patient dosimetry information based on the parameter information along with medical records for the patient, including details such as age and gender, received at the processing component 120 from an HIS.

In an embodiment, the processing component 120 is capable of transforming some or all of the patient dosimetry information. The transformation may include changing the representation or units of measurement of the dosimetry data, for example. The patient dosimetry information may be transformed into a selected representation, for example. The selected representation may be selected by, for example, a user or be pre-configured by an administrator or physician based at least in part on preferences. For example, a user may desire patient dosimetry information to be displayed in Grays. In an embodiment, dosimetry data is transformed to facilitate analysis. In an embodiment, the processing component 120 is capable of transforming dosimetry data in a manner similar to the transformation performed on patient dosimetry information. In an embodiment, the transformation is performed by a transform component.

In an embodiment, the processing component 120 is capable of analyzing patient dosimetry information. The analysis may include, for example, determining statistics or calculating dosages. For example, statistics based at least in part on the dosimetry history may be computed. Statistics may include, for example, lifetime radiation dosage for the patient, an average annual dosage rate, and/or the effective dosage administered to a specific body part or the equivalent dosage for the patient. In an embodiment, the patient dosimetry information has been transformed and/or is the result of transformation, as described above. For example, patient dosimetry information may be transformed into a representation such as Grays to facilitate analysis by the analysis component 224. In an embodiment, the analysis is performed by an analysis component.

In an embodiment, the processing component 120 is capable of providing decision support. For example, the decision support may take the form of a recommendation. The recommendation may be, for example, a procedure or parameters for a procedure to perform. As another example, the recommendation may be a suggested course of treatment or a ranking of suggested courses of treatment.

The decision support may be based at least in part on the patient dosimetry information, such as patient dosimetry history, gender, profession, age, and/or symptoms. In an embodiment, the decision support may be based at least in part on prior cases and/or evidence-based medicine. In an embodiment, the decision support is based at least in part on an analysis result. The analysis result may be from the analysis of patient dosimetry information, described above, for example. In an embodiment, the decision support is performed by a decision support component.

The display 130 may be one or more of a computer screen, a portable computer, a tablet computer, a PACS workstation, a personal digital assistant (PDA), a television, and/or other display, for example. In an embodiment, the display 130 is capable of displaying and/or presenting the patient dosimetry information. In an embodiment, the display 130 is capable of displaying and/or presenting some or all of the dosimetry data. In an embodiment, the display and/or presentation of patient dosimetry information and/or dosimetry data is based at least in part on results from an analysis component. In an embodiment, the display and/or presentation of patient dosimetry information and/or dosimetry data is based at least in part on a recommendation from an decision support component.

The components and/or functionality of system 100 may be implemented alone or in combination in hardware, firmware, and/or as a set of instructions in software, for example. Certain embodiments may be provided as a set of instructions residing on a computer-readable medium, such as a memory, CD, DVD, or hard disk, for execution on a general purpose computer or other processing device, such as, for example, a PACS workstation or image viewer.

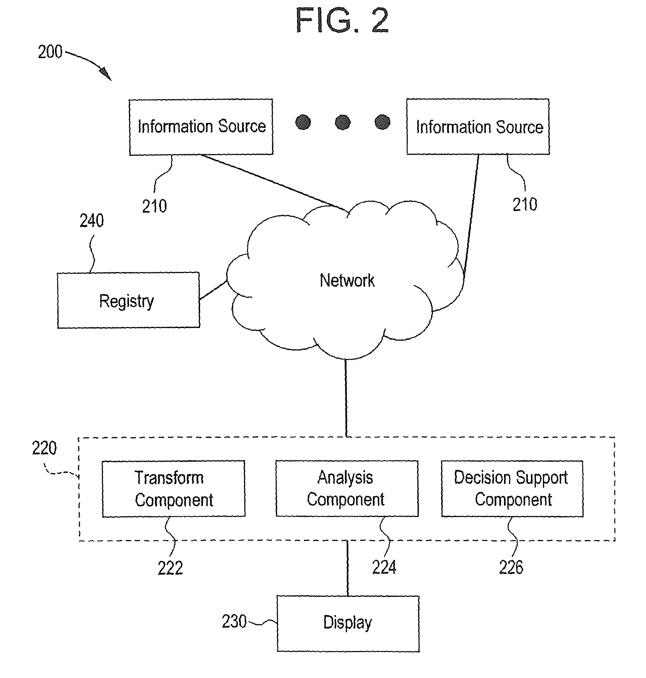

Figure 2 illustrates a system 200 for patient dosimetry management used in accordance with an embodiment of the present invention. The system 200 includes a plurality of information sources 210, a processing component 220, a display 230, and a registry 240. The registry 240 is in communication with the plurality of information sources 210. The registry 240 is in communication with the processing component 220. The processing component 220 is in communication with the plurality of information sources 210. The processing component 220 is in communication with the display 230.

The information source 210 may be similar to the information source 110, described above, for example. The processing component 220 may be similar to the processing component 120, described above, for example. The display 230 may be similar to the display 130, described above, for example.

The processing component 220 includes a transform component 222, an analysis component 224, and a decision support component 226.

One or more of the information sources 210, processing component 220, display 230, and registry 240 may communicate at least in part over a network. The network may be or may be part of an intranet, the Internet, HIS, RIS, or PACS, for example. For example, an information source 210 and the processing component 220 may be connected to a PACS. The processing component 220 may then receive dosimetry data from the information source 210 through part of the PACS network. As another example, one or more information sources 210 may be connected to a network along with the registry 240 and the processing component 220.

In operation, the registry 240 includes information on one or more information sources 210. The registry may be implemented as one or more of a database, table, other data structure, server, hardware component, and/or software component, for example. The information in the registry 240 may include, for example, the type, name, location, and/or a description or representation of at least part of the dosimetry data included in the information source 210. For example, the registry 240 may include information indicating that an information source is a CT scanner, located in a clinic, with a particular network address, and that the information source includes dosimetry data for patients X, Y, and Z. As another example, the registry 240 may include information indicating that an information source is a database with a particular name, network address, and communication and/or query protocols supported by the database. In an embodiment, the registry 240 is capable of facilitating communication between one or more information sources 210 and processing component 220. In an embodiment, no registry 240 is present. As described herein, the registry 240 at least in part facilitates communication between, for example, one or more information sources 210 and processing component 220. Other mechanisms and/or techniques for discovery, identification, and/or facilitation of communication between components on a network not involving registry 240 would be apparent to one having ordinary skill in the art.

In an embodiment, one or more information sources 210 register information relating to the information source and/or the dosimetry data it includes with the registry 240. In an embodiment, the registry 240 is configured with information for one or more information sources 210. The registry 240 may be configured by an administrator, for example. The registry 240 may query one or more information sources 210 for information about the information source and/or the dosimetry data it includes.

The processing component 220 may identify one or more information sources 210 based at least in part on information received from the registry 240. In an embodiment, the processing component 220 is configured with information for one or more information sources 210. The processing component 220 may be configured by an administrator, for example.

Based at least in part on the information from the registry 240, the processing component 220 receives dosimetry data from one or more information sources 220. In certain embodiments, similar to processing component 120 discussed above, the processing component 220 may receive dosimetry data from one or more information sources 220 by "push" and/or "pull" models.

The processing component 220 is capable of generating patient dosimetry information based at least in part on received dosimetry data. In an embodiment, the processing component 220 is capable of generating patient dosimetry information based at least in part on stored dosimetry data. The processing component 220 may generate patient dosimetry information similar to processing component 120, described above, for example. In an embodiment, the processing component 120 may provide some or all of the dosimetry data to one or more of the transform component 222, the analysis component 224, and the decision support component 226. In an embodiment, the processing component 220 may provide some or all of the generated patient dosimetry information to one or more of the transform component 222, the analysis component 224, and the decision support component 226.

The transform component 222 is capable of transforming patient dosimetry information. The transform component 222 may transform some or all of the patient dosimetry information, for example. The transformation performed by the transform component 222 may be similar to the transformation by processing component 120 discussed above, for example. The transformation may include changing the representation or units of measurement of the dosimetry data, for example. The patient dosimetry information may be transformed into a selected representation, for example. The selected representation may be selected by, for example, a user or be pre-configured by an administrator or physician based at least in part on preferences.

For example, a user may desire patient dosimetry information to be displayed in Grays. In an embodiment, the transform component 222 is capable of transforming dosimetry data in a manner similar to the transformation performed on patient dosimetry information. In an embodiment, the transform component 222 is capable of transforming some or all of the dosimetry data.

The analysis component 224 is capable of analyzing patient dosimetry information. The analysis component 224 may analyze some or all of the patient dosimetry information, for example. The analysis performed by the analysis component 224 may be similar to the analysis by processing component 120 discussed above, for example. The analysis may include, for example, determining statistics or calculating dosages. For example, statistics based at least in part on the dosimetry history may be computed. Statistics may include, for example, lifetime radiation dosage for the patient or an average annual dosage rate. In an embodiment, the analysis component 224 is capable of analysis of some or all of the dosimetry data. In an embodiment, the analysis component 224 analyzes some or all of the transformed patient dosimetry information transformed by the transform component 222, described above. For example, patient dosimetry information may be transformed into a representation such as Grays to facilitate analysis by the analysis component 224.

The decision support component 226 is capable of providing decision support based at least in part on patient dosimetry information. The decision support component 226 may provide decision support based at least in part on some or all of the patient dosimetry information, for example. The decision support provided by the decision support component 226 may be similar to the decision support by processing component 120 described above, for example. In an embodiment, the decision support component 226 provides a recommendation. The recommendation may be similar to the recommendation described above, for example. The recommendation may be, for example, a procedure or parameters for a procedure to perform. As another example, the recommendation may be a suggested course of treatment or a ranking of suggested courses of treatment. In an embodiment, the decision support component 226 is capable of providing decision support based at least in part on some or all of the dosimetry data. In an embodiment, the decision support component 226 provides decision support based at least in part on some or all of the transformed patient dosimetry information transformed by the transform component 222, described above. In an embodiment, the decision support component 226 provides decision support based at least in part on some or all of the analysis provided by the analysis component 224, described above.

The display 230 may be one or more of a computer screen, a portable computer, a tablet computer, a PACS workstation, a PDA, a television, and/or other display, for example. In an embodiment, the display 230 is capable of displaying and/or presenting the patient dosimetry information. In an embodiment, the display 230 is capable of displaying and/or presenting some or all of the dosimetry data. In an embodiment, the display and/or presentation of patient dosimetry information and/or dosimetry data is based at least in part on results from an analysis component. The analysis component may be similar to analysis component 224, described above, for example. In an embodiment, the display and/or presentation of patient dosimetry information and/or dosimetry data is based at least in part on a recommendation from an decision support component. The decision support component may be similar to decision support component 226, described above, for example.

The components and/or functionality of system 200 may be implemented alone or in combination in hardware, firmware, and/or as a set of instructions in software, for example. Certain embodiments may be provided as a set of instructions residing on a computer-readable medium, such as a memory, CD, DVD, or hard disk, for execution on a computer or other processing device, such as, for example, a PACS workstation or image viewer.

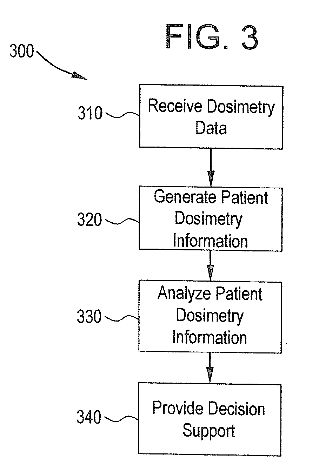

Figure 3 illustrates a flow diagram for a method 300 for patient dosimetry management used in accordance with an embodiment of the present invention. The method 300 includes the following steps, which will be described below in more detail. At step 310, dosimetry data is received. At step 320, patient dosimetry information is generated. At step 330, patient dosimetry information is analyzed. At step 340, decision support is provided. The method 300 is described with reference to elements of systems described above, but it should be understood that other implementations are possible.

At step 310, dosimetry data is received. Dosimetry data may be received from one or more information sources. The information sources may be similar to information source 110 and/or information source 210, described above, for example. In an embodiment, the dosimetry data is received at a processing component. The processing component may be similar to processing component 120 and/or processing component 220, described above, for example. In an embodiment, the processing component aggregates the dosimetry data from one or more information sources 110 and/or 210.

The dosimetry data may be received over a network, for example. The network may be or may be part of an intranet, the Internet, HIS, RIS, or PACS, for example. For example, an information source 110 and the processing component 120 may be connected to a PACS. The processing component 120 may then receive dosimetry data from the information source 110 through part of the PACS network. In an embodiment, a registry is utilized to facilitate communication between one or more information sources (e.g., information sources 110 and/or 210) and the processing component (e.g. processing component 120 and/or 220). The registry may be similar to registry 240, described above, for example.

At step 320, patient dosimetry information is generated. The patient dosimetry information may be generated by a processing component similar to processing component 120 and/or 220, described above, for example. The patient dosimetry information may be generated based at least in part on the dosimetry data received at step 310, described above, for example.

At step 330, patient dosimetry information is analyzed. The patient dosimetry information may be analyzed by an analysis component. The analysis component may be similar to analysis component 224, described above, for example. The analysis may include, for example, determining statistics or calculating dosages. For example, statistics based at least in part on the dosimetry history may be computed.

Statistics may include, for example, lifetime radiation dosage for the patient or an average annual dosage rate.

At step 340, decision support is provided. The decision support may be provided by a decision support component, for example. The decision support component may be similar to decision support component 226, described above, for example. For example, the decision support may take the form of a recommendation. The recommendation may be, for example, a procedure or parameters for a procedure to perform. As another example, the recommendation may be a suggested course of treatment or a ranking of suggested courses of treatment.

In an embodiment, some or all of the dosimetry data and/or patient dosimetry information is transformed. The dosimetry data and/or patient dosimetry information may be transformed by the processing component. The dosimetry data and/or patient dosimetry information may be transformed by a transform component. The transform component may be similar to transform component 222, described above, for example. In an embodiment, the transformed dosimetry data and/or patient dosimetry information is analyzed at step 330, described above. In an embodiment, the decision support provided at step 340, described above, is based at least in part on the transformed dosimetry data and/or patient dosimetry information.

As an example, a referring physician may prescribe a radiology procedure for a patient. The procedure may be scheduled for the radiology department of the appropriate healthcare provider. The scheduled procedure may be communicated to a RIS or PACS. When the scheduled procedure information is received, the RIS or PACS may execute a number of queries to obtain and/or update the longitudinal dosimetry record for the patient based at least in part on received dosimetry data and/or patient dosimetry information. These queries may include, for example, querying the local RIS or PCAS database, looking up references to recent care episodes in a registry, and/or querying remote repositories of dosimetry information for the patient. After receiving the raw dosimetry data and/or patient dosimetry information for the patient, the dosimetry data and/or patient dosimetry information may be consolidated, aggregated, and/or transformed into a consistent and complete dosimetry record. This may include organizing the dosimetry data and/or patient dosimetry information according to date of occurrence and/or translating and/or transforming raw dosimetry data and/or patient dosimetry information into a consistent set of units, for example. The dosimetry record, dosimetry data, and/or patient dosimetry information may be analyzed and/or have statistics computed for total effective radiation dose that the patient has received from medical procedures and typical environmental exposure, for example. As another example, analysis may include a determination of the effective radiation dose for a particular body part, based at least in part upon the relevance of the body part to a scheduled procedure. Subsequent to aggregation and/or analysis of dosimetry data and/or patient dosimetry information and prior to performing a procedure, a radiologist may define or refine the protocol for the procedure using a RIS system, for example. The RIS system may provide the ability to display the longitudinal dosimetry record, dosimetry data, and/or patient dosimetry information for the patient as well as relevant analysis results as described above, for example. An information source may be queried for medical evidence related to the current procedure, disease, patient demographics, and/or patient dosimetry information to provide relevant outcomes information to the radiologist, for example. Protocol recommendations for the current procedure may be provided based at least in part on the aggregate of relevant information include patient dosimetry information, for example. A radiologist may define and/or refine a protocol based on the presentation of the aggregate of information including, for example, longitudinal dosimetry record, dosimetry data, patient dosimetry information, analysis results, relevant medical evidence, and/or clinical recommendations that have been provided.

One or more of the steps of the method 300 may be implemented alone or in combination in hardware, firmware, and/or as a set of instructions in software, for example. Certain embodiments may be provided as a set of instructions residing on a computer-readable medium, such as a memory, CD, DVD, or hard disk, for execution on a general purpose computer or other processing device, such as, for example, a PACS workstation or image viewer.

Certain embodiments of the present invention may omit one or more of these steps and/or perform the steps in a different order than the order listed. For example, some steps may not be performed in certain embodiments of the present invention. As a further example, certain steps may be performed in a different temporal order, including simultaneously, than listed above.

While the invention has been described with reference to certain embodiments, it will be understood by those skilled in the art that various changes may be made and equivalents may be substituted without departing from the scope of the invention. In addition, many modifications may be made to adapt a particular situation or material to the teachings of the invention without departing from its scope. Therefore, it is intended that the invention not be limited to the particular embodiment disclosed, but that the invention will include all embodiments falling within the scope of the appended claims.

### PARTS LIST

| | |
|---|---|
| System for Patient Dosimetry Management | 100 |
| Information Source | 110 |
| Processing Component | 120 |
| Display | 130 |
| System for Patient Dosimetry Management | 200 |
| Information Source | 210 |
| Processing Component | 220 |
| Transform Component | 222 |
| Analysis Component | 224 |
| Decision Support Component | 226 |
| Display | 230 |
| Registry | 240 |
| Flow Diagram | Fig. 3 |

## Claims

1. A system (100, 200) for patient dosimetry management, the system (100, 200) including:
a processing component (120, 220), wherein the processing component (120, 220) is capable of receiving and aggregating dosimetry data from a plurality of information sources (110, 210), wherein each information source (110, 210) in the plurality of information sources (110, 210) includes dosimetry data for a patient, wherein the processing component (120, 220) is capable of generating patient dosimetry information based at least in part on the aggregated dosimetry data.

2. The system (100, 200) of claim 1, wherein the processing component (120, 220) is capable of transforming the dosimetry data into a selected representation.

3. The system (100, 200) of claim 1 or claim 2, wherein the processing component (120, 220) is capable of analyzing the patient dosimetry information.

4. The system (100, 200) of any preceding claim, wherein the processing component (120, 220) is capable of providing decision support based at least in part on the patient dosimetry information.

5. The system (100, 200) of any preceding claim, wherein the processing component (120, 220) requests at least part of the dosimetry data from at least one information source (110, 210) in the plurality of information sources (110, 210).

6. The system (100, 200) of any preceding claim, wherein at least one information source (110, 210) in the plurality of information sources (110, 210) provides at least part of the dosimetry data to the processing component (120, 220) when new dosimetry data is available.

7. The system (100, 200) of any preceding claim, wherein the processing component (120, 220) generates the patient dosimetry information based at least in part on stored dosimetry data.

8. A method for patient dosimetry management, the method including:
receiving dosimetry data at a processing component (120, 220), wherein the dosimetry data is received from a plurality of information sources (110, 210); and generating patient dosimetry information based at least in part on the dosimetry data.

9. The method of claim 8, further including utilizing a registry (240) to facilitate communication between the processing component (120, 220) and at least one information source (110, 210) in the plurality of information sources (110, 210).

10. The method of claim 8 or claim 9, further including providing a recommendation based at least in part on the patient dosimetry information.
